# EUROPÄISCHE PATENTSCHRIFT

(11) **EP 3 201 127 B1**
(45) Veröffentlichungstag und Bekanntmachung des Hinweises auf die Patenterteilung: **05.09.2018**
(21) Anmeldenummer: 16702066.8
(22) Anmeldetag: 26.01.2016
(51) Int. Cl.: C01B 7/07, C07C 17/16

(54) **VERFAHREN ZUR REINIGUNG VON VERUNREINIGTEM GASFÖRMIGEM CHLORWASSERSTOFF**
METHOD FOR PURIFYING CONTAMINATED GASEOUS HYDROGEN CHLORIDE
PROCÉDÉ DE PURIFICATION D'ACIDE CHLORHYDRIQUE GAZEUX IMPUR

(30) Priorität: 28.01.2015 DE 102015201446
(43) Veröffentlichungstag der Anmeldung: 09.08.2017
(73) Patentinhaber: Wacker Chemie AG, 81737 München (DE)
(72) Erfinder: MAUTNER, Konrad, 84489 Burghausen (DE)
(74) Vertreter: Fritz, Helmut
(86) Internationale Anmeldenummer: PCT/EP2016/051606
(87) Internationale Veröffentlichungsnummer: WO 2016/120288

(56) Entgegenhaltungen:
- EP-A1- 0 342 521
- CN-A- 101 423 193
- CN-A- 103 724 367
- JP-A- H05 330 804

## Beschreibung

Die Erfindung betrifft ein Verfahren zur Reinigung von verunreinigtem gasförmigem Chlorwasserstoff mit flüssigem Organochlorsilan.

Organochlorsilanhydrolysen werden technisch häufig so durchgeführt, dass gasförmiger Chlorwasserstoff entsteht.
Dieser Chlorwasserstoff enthält üblicherweise neben Feuchtigkeit auch Spuren von Ausgangssilan und dessen Hydrolyseprodukten. Im Fall von Dimethyldichlorsilan als Ausgangssilan sind neben Wasser noch Spuren cyclischer Siloxane z.B. Hexamethylcyclotrisiloxan und Octamethylcyclotetrasiloxan, lineare Oligosiloxane, z.B. Dichlortetramethyldisiloxan oder Dihydroxyteramethyldisiloxan und höhere chlor- oder hydroxyentständige Homologe enthalten.

Der Chlorwasserstoff wird beispielsweise in Siloxananlagen zusammen mit Methanol zur Herstellung von Chlormethan eingesetzt: MeOH + HCl --> MeCl + H₂O (Me = Methyl).
Gelangen diese Siloxanbestandteile in eine Chlormethananlage, dann wird bei Gasphasenkatalysatoren, meist aktivierte Aluminiumoxide, der Katalysator durch Verlegung der Oberfläche inaktiv. Bei Flüssigphasenkatalyse kann es zu Fouling bei Wärmetauschern führen und die Siloxanbestandteile gelangen mit dem Reaktionswasser ins Abwasser und führen dort zu CSB-Belastung.

Bevorzugt wird der Chlorwasserstoff deshalb vor der Chlormethanherstellung von Silan- und Siloxanbestandteilen befreit.

DE 3816783A1 beschreibt die destillative Aufreinigung von verunreinigtem Chlorwasserstoff. Das ist sehr aufwendig, da Chlorwasserstoff aufgrund des sehr niedrigen Siedepunkts komprimiert werden muss, um den gereinigten Chlorwasserstoff kondensieren zu können.

CN 101423193 A und CN 203333289 beschreiben eine Wäsche mit kalter Salzsäure, die bevorzugt aus dem Hydrolyseprozess stammt. Dazu muss aber die Salzsäure mit zusätzlichen, technischen Einrichtungen auf Temperaturen unter - 15 °C abgekühlt werden.

CN103724367A beschreibt eine Wäsche mit Chlormethan als Teil eines komplexen Reinigungssystems. Die angegebenen tiefen Temperaturen bergen die Gefahr von Vereisung durch Wasser und/oder feste Siloxanbestandteile, beispielsweise liegt der Fp. von Octamethylcyclotetrasiloxan bei 17 - 18 ° C. Bei Anwesenheit von Feuchtigkeit sind korrosionsbeständige Werkstoffe für sehr tiefe Temperaturen (< -40 °C) nötig, was die Werkstoffauswahl stark kompliziert.

Gegenstand der Erfindung ist ein Verfahren zur Reinigung von verunreinigtem gasförmigem Chlorwasserstoff, der Verunreinigungen enthält, die ausgewählt werden aus Wasser, Organochlorsilan A und dessen Hydrolyseprodukten, bei dem der gasförmige Chlorwasserstoff mit flüssigem Organochlorsilan B gewaschen wird und nach der Wäsche mit Organochlorsilan B mit flüssigem Chlormethan gewaschen wird.

Das Verfahren ist einfach und kostengünstig durchzuführen. Die Verunreinigungen werden zuverlässig entfernt. Bei der Handhabung des gewaschenen gasförmigen Chlorwasserstoffs besteht keine Gefahr von Vereisungen, es können die für die Handhabung von trockenem gasförmigem Chlorwasserstoff üblichen Werkstoffe eingesetzt werden und es ist nur ein geringerer Einsatz an Kältemittel notwendig.

Vorzugsweise stammt der gasförmige Chlorwasserstoff aus der Hydrolyse von Organochlorsilanen A. Das Verfahren ist grundsätzlich anwendbar auf alle Hydrolysen von Organochlorsilanen A, die gasförmigen Chlorwasserstoff liefern.

Die Organochlorsilane A weisen vorzugsweise die allgemeine Formel I

RₐSiCl₄₋ₐ (I)

auf, in der
- **R**: gleiche oder verschieden Reste, ausgewählt aus Wasserstoff und unsubstituierten und substituierten C₆ bis C₁₈ Kohlenwasserstoffresten bedeutet und
- **a**: Werte 0, 1, 2 oder 3 bedeutet,
mit der Massgabe, dass mindestens 10 mol-% der Reste **R** Kohlenwasserstoffreste sind.

Beispiele für Reste **R** in der allgemeinen Formel (I) sind Alkylreste, wie der Methyl-, Ethyl-, n-Propyl-, iso-Propyl-, n-Butyl-, iso-Butyl-, tert.-Butyl-, n-Pentyl-, iso-Pentyl-, neoPentyl-, tert.-Pentylrest, Hexylreste, wie der n-Hexylrest, Heptylreste, wie der n-Heptylrest, Octylreste, wie der n-Octylrest und iso-Octylreste, wie der 2,2,4-Trimethylpentylrest, Nonylreste, wie der n-Nonylrest, Decylreste, wie der n-Decylrest; Cycloalkylreste, wie Cyclopentyl-, Cyclohexyl-, 4-Ethylcyclohexyl-, Cycloheptylreste, Norbornylreste und Methylcyclohexylreste; Arylreste, wie der Phenyl-, Biphenylyl-, Naphthylrest; Alkarylreste, wie o-, m-, p-Tolylreste und Ethylphenylreste; Aralkylreste, wie der Benzylrest, der alpha- und der ß-Phenylethylrest.

Beispiele für substituierte Reste **R** sind Halogenalkylreste, wie der 3,3,3-Trifluor-n-propylrest, der 2,2,2,2',2',2'-Hexafluorisopropylrest und der Heptafluorisopropylrest.

**R** weist bevorzugt 1 bis 6 Kohlenstoffatome auf. Insbesondere bevorzugt sind Methyl- oder Phenylrest.

Besonders bevorzugt werden Dimethyldichlorsilan, Trimethylchlorsilan, Methyltrichlorsilan und Gemische davon hydrolysiert. Insbesondere stammt der gasförmige Chlorwasserstoff aus der Hydrolyse von Dimethyldichlorsilan. Vorzugsweise wird der verunreinigte gasförmige Chlorwasserstoff mit dem Ausgangschlorsilan der Hydrolyse gewaschen, das bedeutet, daß Organochlorsilan A und Organochlorsilan B identisch sind.

Besonders bevorzugt ist Organochlorsilan B Dimethyldichlorsilan, Trimethylchlorsilan, Methyltrichlorsilan und Gemische davon. Insbesondere ist Organochlorsilan B Dimethyldichlorsilan.

Vorzugsweise wird zum Waschen mindestens die stöchiometrisch 10 fache, insbesondere 20 fache Menge an Oganochlorsilan B eingesetzt, die zum Hydrolysieren des Wassers des verunreinigtem gasförmigem Chlorwasserstoffs erforderlich ist. Damit werden Wasser vollständig und höhersiedende Oligomere größtenteils entfernt.

Der Chlorwasserstoff ist nach dem Waschen mit flüssigem Organochlorsilan B entsprechend Druck und Temperatur mit dem Organochlorsilan B gesättigt. Der Vorteil besteht darin, dass nun eine einfache Werkstoffauswahl für weitere Verarbeitungsschritte erfolgen kann, da der Chlorwasserstoff nicht mehr korrosiv wirkt und ein anderer Vorteil liegt darin, dass die Organochlorsilane A und B meist sehr niedrige Schmelzpunkte besitzen und deswegen auch bei sehr tiefen Temperaturen nicht mit Vereisung zu rechnen ist. Für den bevorzugten Fall von Dimethyldichlorsilan beträgt der Schmelzpunkt -76°C. Nach diesem Schritt können einfache, metallische Werkstoffe verwendet werden, die günstiger und auch weniger empfindlich sind gegenüber mechanischer Belastung.

Die Wäsche des Chlorwasserstoffs mit Organochlorsilan B wird vorzugsweise bei Temperaturen von 0°C bis 50°C, insbesondere 10°C bis 30°C durchgeführt. Vorzugsweise betragen die Drucke 1 bis 20 bar, insbesondere 2 bis 10 bar.

Die Wäsche des Chlorwasserstoffs mit Organochlorsilan B wird vorzugsweise in einer Waschkolonne durchgeführt. Die Waschkolonne besteht vorzugsweise aus Glas, emailliertem Metall oder Kunststoff.

Der Chlorwasserstoff wird nach der Wäsche mit Organochlorsilan B mit flüssigem Chlormethan gewaschen. Das nun trockene Chlorwasserstoffgas wird durch die Verdampfung des Chlormethans abgekühlt. Die Temperatur beträgt dabei vorzugsweise -15 °C bis -55°C, besonders bevorzugt -20 °C bis -50 °C. Vorzugsweise betragen die Drucke 1 bis 20 bar, insbesondere 2 bis 10 bar. Dabei werden die Silanbestandteile auskondensiert und diese können in den ersten Wäscher zurückgeführt werden. Die Chlormethanmenge wird vorzugsweise so geregelt, dass am Kopf der Waschkolonne eine konstante, gewünschte Temperatur erzielt wird. Der gereinigte Chlorwasserstoff enthält dampfdruckgemäß Chlormethan. Da der Chlorwasserstoff jedoch bevorzugt zur Herstellung von Chlormethan verwendet wird, stört dieser Bestandteil bei dieser Weiterverwendung nicht und kann deshalb im Chlorwasserstoff verbleiben. Vorzugsweise wird der gasförmige Chlorwasserstoff als Rohstoff für die Chlormethansynthese eingesetzt.

Vorzugsweise wird zum Waschen mit Chlormethan die 0,05 fache bis 1 fache, insbesondere die 0,1 fache bis 0,5 fache Menge an Chlormethan eingesetzt, jeweils bezogen auf die Masse des gasförmigen Chlorwasserstoffs.

Die Wäsche des Chlorwasserstoffs mit Chlormethan wird vorzugsweise in einer Waschkolonne durchgeführt. Vorzugsweise wird in der Waschkolonne das flüssige Chlormethan im oberen Drittel der Kolonne beaufschlagt. Die Waschkolonne kann aus Metall, insbesondere Stahl bestehen.

Alle vorstehenden Symbole der vorstehenden Formeln weisen ihre Bedeutungen jeweils unabhängig voneinander auf. Soweit nicht anders erwähnt sind alle Mengen - und Prozentangaben auf das Gewicht bezogen, alle Drücke 0,10 MPa (abs.) und alle Temperaturen 20°C.

### Beispiel:

Die folgenden Massenangaben wurden bestimmt durch Gaschromatographie.

Ein HCl-Strom aus einer Hydrolyse von Dimethyldichlorsilan enthält 0,15 % Hexamethylcyclotetrasiloxan 0,32 % Octamethylcyclotetrasiloxan, 0,06 % Dekamethylcyclopentasiloxan, 0,15 % Dichlortetramethyldisiloxan und 0,5 % Wasser.

In einem ersten Wäscher werden bei 4 bar 17 t/h dieses verunreinigten HCl-Gases am unteren Ende zugegeben und am Kopf mit 500 kg Dimethyldichlorsilan beaufschlagt, dazu kommen 1000 kg Sumpf aus einem Chlormethan Wäscher. Wasser und Siloxane werden bis unter die Nachweisgrenze eliminiert.

Das Kopfprodukt des ersten Wäschers wird am unteren Ende in den Chlormethan Wäscher zugegeben, dabei gelangen 1000 kg/h Dimethyldichlorsilan in den Chlormethan Wäscher, der am Kopf mit 2600 kg/h Chlormethan flüssig beaufschlagt wird. Es stellen sich dort Temperaturen von -44 °C am Kopf und -20 °C im Sumpf ein. Das Dimethyldichlorsilan wird im Chlormethan Wäscher als Sumpf vollständig abgetrennt und auf den ersten Wäscher zurückgeführt. Das am Kopf des Chlormethan Wäschers abgeführte von Siloxankomponenten gereinigte HCl-Gas wird mit einem Anteil 2600 kg/h Chlormethan einer Chlormethan-Synthese zugeführt.

## Patentansprüche

1. Verfahren zur Reinigung von verunreinigtem gasförmigem Chlorwasserstoff, der Verunreinigungen enthält, die ausgewählt werden aus Wasser, Organochlorsilan A und dessen Hydrolyseprodukten, bei dem der gasförmige Chlorwasserstoff mit flüssigem Organochlorsilan B gewaschen wird und nach der Wäsche mit Organochlorsilan B mit flüssigem Chlormethan gewaschen wird.

2. Verfahren nach Anspruch 1, bei dem der gasförmige Chlorwasserstoff aus der Hydrolyse von Organochlorsilanen A stammt.

3. Verfahren nach einem oder mehreren der vorangehenden Ansprüche, bei dem die Organochlorsilane A ausgewählt werden aus Dimethyldichlorsilan, Trimethylchlorsilan, Methyltrichlorsilan und Gemischen davon.

4. Verfahren nach einem oder mehreren der vorangehenden Ansprüche, bei dem Organochlorsilan A und Organochlorsilan B identisch sind.

5. Verfahren nach einem oder mehreren der vorangehenden Ansprüche, bei dem zum Waschen mindestens die stöchiometrisch 10 fache Menge an Oganochlorsilan B eingesetzt wird, die zum Hydrolysieren des Wassers des verunreinigtem gasförmigem Chlorwasserstoffs erforderlich ist.

6. Verfahren nach einem oder mehreren der vorangehenden Ansprüche, bei dem die Wäsche des Chlorwasserstoffs mit Organochlorsilan B bei Temperaturen von 0°C bis 50°C durchgeführt wird.

7. Verfahren nach nach einem oder mehreren der vorangehenden Ansprüche, bei dem die Temperatur bei der Wäsche mit flüssigem Chlormethan -15 °C bis -55°C beträgt.

8. Verfahren nach einem oder mehreren der vorangehenden Ansprüche, bei dem der gereinigte Chlorwasserstoff zur Herstellung von Chlormethan verwendet wird.

## Claims

1. Process for purifying contaminated gaseous hydrogen chloride containing impurities selected from among water, organochlorosilane A and hydrolysis products thereof, wherein the gaseous hydrogen chloride is scrubbed with liquid organochlorosilane B and is scrubbed with liquid chloromethane after the scrub using organochlorosilane B.

2. Process according to Claim 1, wherein the gaseous hydrogen chloride originates from the hydrolysis of organochlorosilanes A.

3. Process according to one or more of the preceding claims, wherein the organochlorosilanes A are selected from among dimethyldichlorosilane, trimethylchlorosilane, methyltrichlorosilane and mixtures thereof.

4. Process according to one or more of the preceding claims, wherein organochlorosilane A and organochlorosilane B are identical.

5. Process according to one or more of the preceding claims, wherein at least 10 times the stoichiometric amount of organochlorosilane B required for hydrolyzing the water of the contaminated gaseous hydrogen chloride is used for scrubbing.

6. Process according to one or more of the preceding claims, wherein the scrubbing of the hydrogen chloride using organochlorosilane B is carried out at temperatures of from 0°C to 50°C.

7. Process according to one or more of the preceding claims, wherein the temperature in the scrub using liquid chloromethane is from -15°C to -55°C.

8. Process according to one or more of the preceding claims, wherein the purified hydrogen chloride is used for preparing chloromethane.

## Revendications

1. Procédé de purification de chlorure d'hydrogène gazeux contaminé, qui contient des impuretés qui sont choisies parmi l'eau, un organochlorosilane A et ses produits d'hydrolyse, selon lequel le chlorure d'hydrogène gazeux est lavé avec un organochlorosilane liquide B et lavé avec du chlorométhane liquide après le lavage avec l'organochlorosilane B.

2. Procédé selon la revendication 1, selon lequel le chlorure d'hydrogène gazeux est issu de l'hydrolyse d'organochlorosilanes A.

3. Procédé selon une ou plusieurs des revendications précédentes, selon lequel les organochlorosilanes A sont choisis parmi le diméthyldichlorosilane, le triméthylchlorosilane, le méthyltrichlorosilane et leurs mélanges.

4. Procédé selon une ou plusieurs des revendications précédentes, selon lequel l'organochlorosilane A et l'organochlorosilane B sont identiques.

5. Procédé selon une ou plusieurs des revendications précédentes, selon lequel au moins 10 fois la quantité stoechiométrique d'organochlorosilane B nécessaire pour l'hydrolyse de l'eau du chlorure d'hydrogène gazeux contaminé est utilisée pour le lavage.

6. Procédé selon une ou plusieurs des revendications précédentes, selon lequel le lavage du chlorure d'hydrogène avec l'organochlorosilane B est réalisé à des températures de 0 °C à 50 °C.

7. Procédé selon une ou plusieurs des revendications précédentes, selon lequel la température lors du lavage avec le chlorométhane liquide est de -15 °C à -55 °C.

8. Procédé selon une ou plusieurs des revendications précédentes, selon lequel le chlorure d'hydrogène purifié est utilisé pour la fabrication de chlorométhane.
